# FASCICULE DE BREVET EUROPEEN

(11) **EP 2 931 338 B1**
(45) Date de publication et mention de la délivrance du brevet: **30.11.2016**
(21) Numéro de dépôt: 13821866.4
(22) Date de dépôt: 11.12.2013
(51) Int. Cl.: A61M 5/32, A61M 5/31

(54) **DISPOSITIF D'INJECTION COMPORTANT UN SYSTÈME DE PROTECTION D'AIGUILLE**
INJEKTIONSVORRICHTUNG MIT EINER SCHUTZVORRICHTUNG FÜR EINE SPRITZENNADEL
INJECTION DEVICE WITH NEEDLE PROTECTION SYSTEM

(30) Priorité: 14.12.2012 FR 1262066
(43) Date de publication de la demande: 21.10.2015
(73) Titulaire: Aptar Stelmi SAS, 93420 Villepinte (FR)
(72) Inventeur: FOURNIER, Arnaud, F-75007 Paris (FR); FOURNIER, Ghislain, F-17000 La Rochelle (FR); SWAL, Mickaël, F-77124 Chauconin Neufmontiers (FR)
(74) Mandataire: CAPRI
(86) Numéro de dépôt international: PCT/FR2013/053030
(87) Numéro de publication internationale: WO 2014/091153

(56) Documents cités:
- EP-A1- 0 518 416
- EP-A1- 1 208 861
- WO-A1-2004/110535
- WO-A1-2009/081103
- WO-A1-2009/087355
- WO-A1-2012/164397
- WO-A2-00/09186
- DE-A1-102008 003 185
- FR-A1- 2 777 787
- US-A- 4 986 818

## Description

La présente invention concerne un dispositif d'injection comportant un système de protection d'aiguille.

Les dispositifs d'injection sont bien connus, et comportent généralement un corps dans lequel est disposée une seringue contenant un produit fluide à distribuer, ledit corps incorporant des moyens de distribution adaptés à déplacer le piston de la seringue pour ainsi réaliser l'injection. Un type de dispositif d'injection bien connu comporte les autoinjecteurs, dans lesquels l'injection est réalisée automatiquement au moyen d'un ou plusieurs ressort(s). Dans ce type de dispositif d'injection, l'aiguille de la seringue doit, avant toute utilisation, être protégée et notamment maintenue stérile.

A cet effet, il est connu d'utiliser un protège-aiguille amovible, tel que par exemple décrit dans les documents FR-2 777 787 et FR-2 816 848. Ce type de protège-aiguille comporte généralement une partie d'isolation en matériau souple dans laquelle le bout de l'aiguille incorporant l'orifice de distribution est piqué dans une position de protection ou de non-distribution. Lorsque l'utilisateur souhaite utiliser le dispositif d'injection, il retire manuellement le protège-aiguille pour libérer l'aiguille et ainsi permettre l'injection du produit.

Un problème qui se pose avec ce type de dispositif concerne la sécurité des personnes qui manipulent le dispositif d'injection, et notamment le risque de piqûre par l'aiguille lorsque le protège-aiguille a été retiré ou pendant la phase du retrait dudit protège-aiguille. Par ailleurs, notamment dans le cadre des autoinjecteurs, le retrait du protège-aiguille de sa position de protection ou de non-distribution doit être aussi fiable et simple que possible.

Les documents WO 2009/081103, WO 2012/164397, FR 2 777 787, EP 0 518 416, WO 00/09186, US 4 986 818, WO 2004/110535, WO2009/87355 et DE 10 2008 003185 décrivent des dispositifs de l'art antérieur.

La présente invention a pour but de fournir un dispositif d'injection comportant un système de protection d'aiguille qui ne reproduit pas les inconvénients susmentionnés.

La présente invention a notamment pour but de fournir un tel système de protection qui soit simple et peu coûteux à fabriquer et à assembler et fiable d'utilisation.

La présente invention a donc pour objet un dispositif d'injection comportant un système de protection d'aiguille, ledit dispositif d'injection comportant une aiguille de distribution pourvue d'un orifice de distribution, ledit système de protection comportant un protège-aiguille amovible adapté à être disposé, dans une position de non-distribution, autour de ladite aiguille pour protéger et isoler ledit orifice de distribution de ladite aiguille, ledit protège-aiguille comportant un corps latéral creux et une partie d'isolation, au moins ladite partie d'isolation étant en matériau souple, ledit orifice de distribution de l'aiguille étant piqué dans ladite partie d'isolation en position de non-distribution, ledit protège-aiguille comportant au moins une projection radiale vers l'extérieur, ledit système de protection comportant un capot externe pourvu de moyens de préhension adaptés à coopérer avec ladite au moins une projection radiale dudit protège-aiguille, de telle sorte qu'un déplacement dudit capot externe dans le sens longitudinal de ladite aiguille en éloignement dudit orifice de distribution retire ledit protège-aiguille de sa position de non-distribution, ladite au moins une projection radiale étant disposée à l'extrémité axiale externe dudit protège-aiguille, ladite au moins une projection radiale étant formée sur une structure externe rigide dudit protège-aiguille.

Avantageusement, ladite au moins une projection radiale est formée par un disque définissant une projection radiale périphérique.

Avantageusement, lesdits moyens de préhension comportent au moins un épaulement radial s'étendant radialement vers l'intérieur dudit capot externe et coopérant, notamment par encliquetage, avec ladite au moins une projection radiale dudit protège-aiguille.

Avantageusement, ledit au moins un épaulement radial est formé par une bride radiale périphérique ou par plusieurs parties de bride radiale réparties sur la périphérie.

Avantageusement, ledit au moins un épaulement est formé par une patte déformable.

Avantageusement, lesdits moyens de préhension comportent une surface interne conique coopérant par coincement et/ou frottements avec le bord radialement externe de ladite au moins une projection radiale.

Avantageusement, lesdits moyens de préhension sont formés de manière monobloc avec ledit capot.

En variante, lesdits moyens de préhension sont formés sur une pièce interne mobile par rapport audit capot.

Avantageusement, une bague dudit dispositif d'injection coopère avec ledit protège-aiguille en position de non-distribution pour former une butée mécanique lors de la mise en place du capot externe autour dudit protège-aiguille.

Avantageusement, ledit dispositif d'injection est un autoinjecteur.

Ces caractéristiques et avantages et d'autres de la présente invention apparaitront plus clairement au cours de la description détaillée suivante, faite en référence aux dessins joints, donnés à titre d'exemples non limitatifs, et sur lesquels
La figure 1 est une vue schématique en perspective partiellement découpée d'un dispositif d'injection incorporant un système de protection d'aiguille selon un premier mode de réalisation de la présente invention, avec le protège-aiguille retiré,
La figure 2 est une vue schématique en section transversale d'une partie du dispositif représenté sur la figure 1,
La figure 3 est une vue similaire à celle de la figure 1, montrant un second mode de réalisation de la présente invention,
Les figures 4 et 5 représentent de manière schématique et en perspective partiellement découpée une variante de réalisation de la présente invention, respectivement en position non-assemblée et en position assemblée,
Les figures 6 et 7 sont des vues similaires aux figures 4 et 5, représentant une autre variante de réalisation de la présente invention,
Les figures 8 et 9 sont des vues similaires à celles des figures 4 et 5, représentant encore une autre variante de réalisation de la présente invention,
Les figures 10 et 11 sont des vues similaires à celles des figures 4 et 5, représentant encore une autre variante de réalisation de la présente invention,
Les figures 12 à 14 sont des vues schématiques, représentant encore une autre variante de réalisation de la présente invention,
Les figures 15 et 16 sont des vues schématiques, représentant encore une autre variante de réalisation de la présente invention,
La figure 17 est une vue schématique en section transversale d'un autre mode de réalisation, qui n'est pas couvert par la présente invention, en position de non-distribution, et
La figure 18 est une vue similaire à celle de la figure 17, prise selon un autre angle de vue.

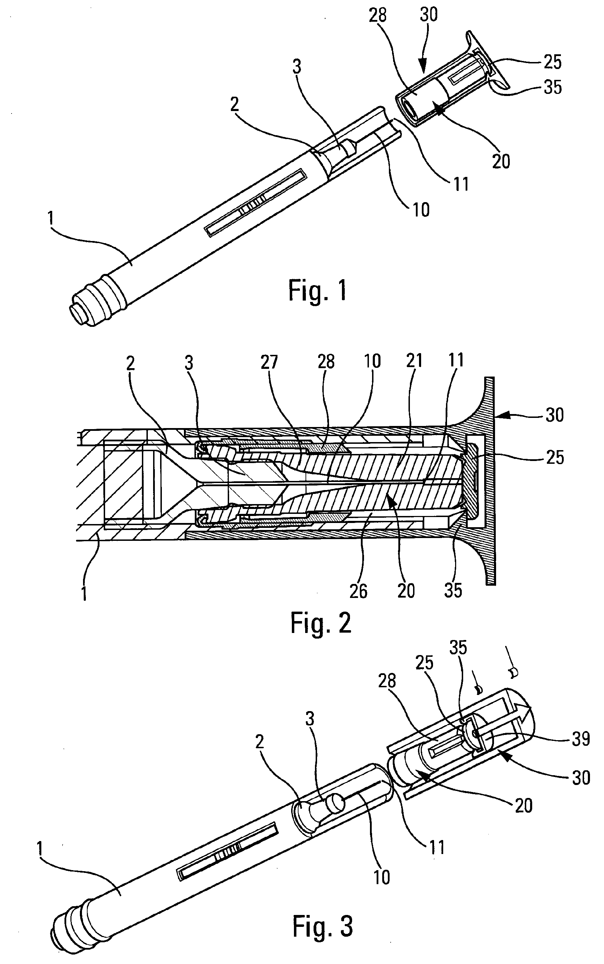

Les figures 1 et 2 représentent un premier mode de réalisation de l'invention. Celui-ci illustre de manière très schématique un dispositif d'injection tel qu'un autoinjecteur. Cet autoinjecteur comporte un corps externe 1 qui peut contenir une seringue 2 formant un réservoir contenant le produit à injecter. Ladite seringue 2 est pourvue d'une extrémité avant 3 munie d'une aiguille 10, ladite aiguille ayant un orifice de distribution 11 à son extrémité axiale distale, c'est-à-dire son extrémité axiale la plus éloignée dudit réservoir.

Un protège-aiguille amovible 20 est prévu pour recouvrir ladite aiguille 10 dans une position de non-distribution qui est représentée sur la figure 2. Ce protège-aiguille 20 peut comporter de manière connue une partie d'isolation 21 en matériau souple dans laquelle l'orifice de distribution 11 de l'aiguille 10 est piqué en position de non-distribution, pour isoler ainsi ladite guille et donc maintenir le contenu de la seringue 2 stérile. De manière connue, ledit protège-aiguille 20 peut comporter un corps latéral creux 27, ouvert d'un coté axial proximal où il vient s'emmancher sur l'extrémité avant 3 de la seringue 2, et fermé de l'autre coté axial distal par ladite partie d'isolation 21 en matériau souple. Eventuellement, le corps latéral 27 peut également être réalisé en matériau souple, comme représenté dans les exemples des figures.

Une structure externe 28 en matériau rigide est prévue autour dudit corps latéral 27, notamment pour rigidifier le protège-aiguille 20. Les protèges-aiguilles bi-matières de ce type sont bien connus et sont notamment décrits dans les documents cités précédemment. La partie souple du protège-aiguille peut être réalisée par exemple en caoutchouc ou en TPE (thermoplastique élastomère). La partie rigide peut être réalisée en tout matériau plastique approprié, par exemple en PP (polypropylène). En variante, la partie rigide pourrait aussi être réalisée, en partie ou totalement, en métal, par exemple en acier.

Selon l'invention, le protège-aiguille 20 comporte au moins une projection radiale 25 s'étendant radialement vers l'extérieur. Cette projection 25 peut notamment être réalisée sous la forme d'un disque. Cette projection radiale 25 est fixée ou formée sur l'extrémité axiale distale du protège-aiguille 20, comme représenté sur les figures. En variante, il pourrait y avoir plusieurs projections réparties autour de la périphérie de l'extrémité axiale distale dudit protège-aiguille.

Selon l'invention, et comme représenté dans les exemples des figures 1 à 16, ladite projection radiale 25 est réalisée sur ladite structure externe rigide 28. Cette structure externe rigide 28 peut comporter avantageusement une ou plusieurs fentes latérales 26. Eventuellement, un évidement radial 22 peut être formé entre ladite structure externe rigide 28 et la projection radiale 25.

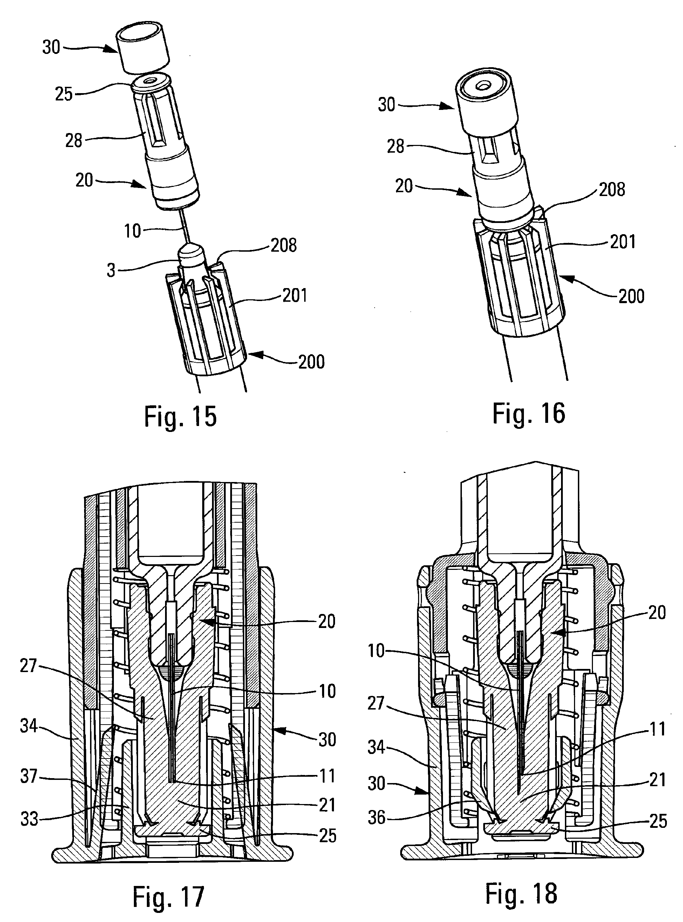

Dans une variante qui n'est pas couverte par l'invention, représentée sur les figures 17 et 18, la projection radiale 25 est formée directement sur ledit corps latéral 27 du protège-aiguille. Cette mise en oeuvre présente l'inconvénient d'avoir à réaliser un protège-aiguille complexe en matériau souple, intégrant ladite projection radiale. De plus, l'épaulement radial étant en matériau souple, il devient déformable, ce qui peut être désavantageux pour son fonctionnement. Dans cette variante, le diamètre extérieur de ladite projection radiale 25 est de préférence supérieur au diamètre extérieur dudit corps latéral 27, comme visible sur les figures 17 et 18. Il est à noter que le mode de réalisation des figures 17 et 18 pourrait s'appliquer à un protège-aiguille avec une structure externe rigide, auquel cas il serait couvert par l'invention.

Le système de protection selon l'invention comporte par ailleurs un capot externe 30 qui vient s'emmancher autour dudit protège-aiguille 20, et qui comporte des moyens de préhension adaptés à coopérer avec ladite au moins une projection radiale distale 25 dudit protège-aiguille 20.

Ces moyens de préhension peuvent avantageusement comporter au moins un épaulement radial s'étendant radialement vers l'intérieur à partir d'une surface interne dudit capot.

Ainsi, en se référant à l'exemple des figures 1 et 2, on voit que cet épaulement radial est formé par une bride périphérique continue 35 se projetant radialement vers l'intérieur à partir de la surface interne du capot externe 30, qui vient s'encliqueter sous ladite projection radiale distale 25 du protège-aiguille.

Lorsque l'utilisateur retire le capot 30 en le déplaçant axialement dans le sens longitudinal de l'aiguille 10 en éloignement de l'orifice de distribution 11, le protège-aiguille 20 est retiré de sa position de non-distribution ou de protection pour ainsi libérer l'aiguille, comme visible sur la figure 1.

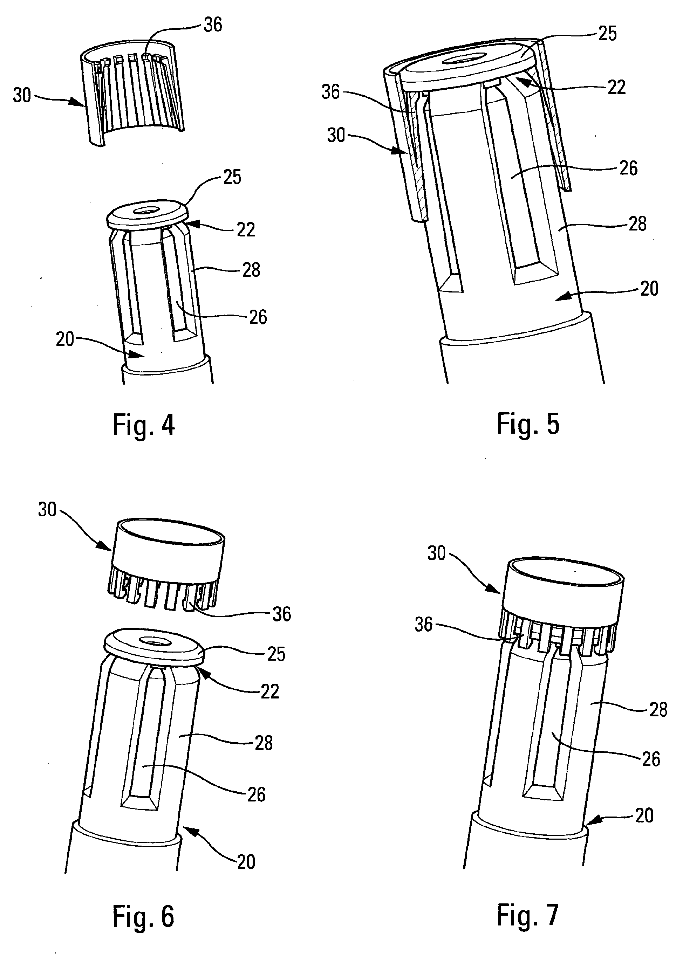

Les figures 4 à 9 illustrent diverses variantes de réalisation dudit au moins un épaulement radial du capot 30.

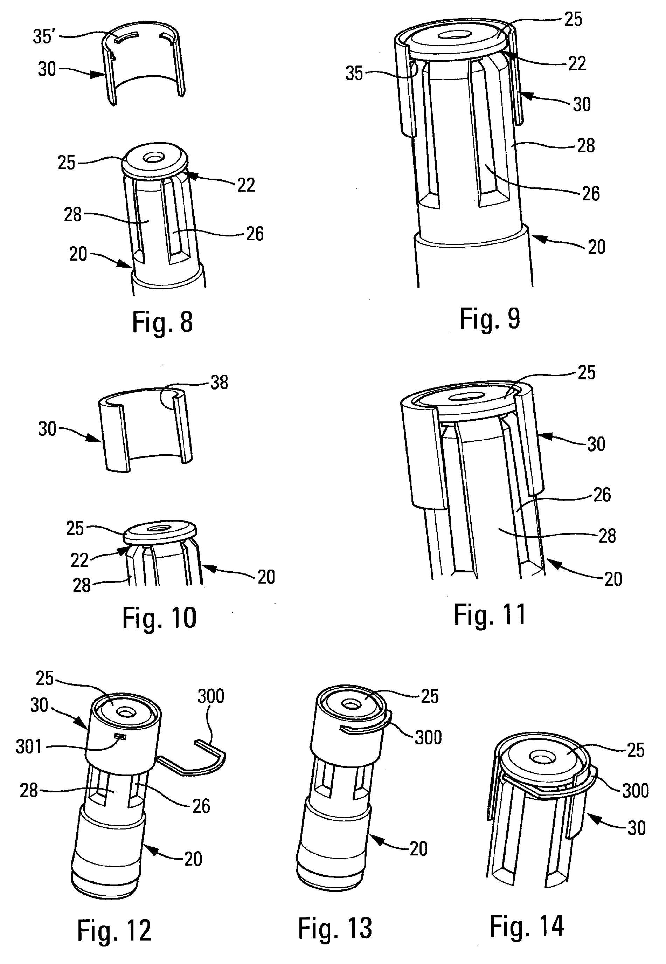

Ainsi, alors que dans la figure 2 cet épaulement est formé par une bride radiale périphérique 35 à l'intérieur du capot 30, on peut très bien prévoir plusieurs parties de bride radiale 35' réparties sur la périphérie, comme représenté sur la figure 8.

En variante, on peut également prévoir des pattes déformables 36, qui peuvent être soit des pattes orientées axialement en direction de ladite projection radiale 25, comme visible sur les figures 4 et 5, soit des pattes d'encliquetage classiques orientées dans le sens inverse, comme représenté sur les figures 6 et 7.

Bien entendu, tous types d'épaulement(s) adapté(s) à coopérer avec tous types de projection(s) sont envisageables dans le cadre de la présente invention pour permettre une coopération entre le capot externe 30 et le protège-aiguille 20.

Les figures 10 et 11 illustrent une variante de réalisation, dans laquelle le capot externe 30 ne comporte pas d'épaulement radial interne, mais une surface interne conique, qui va coopérer par coincement et/ou frottements avec le bord radialement externe de la projection radiale distale 25.

Les figures 12 à 14 montrent encore une autre variante, dans laquelle un organe de préhension externe 300 coopère avec le capot externe 30 et avec ladite au moins une projection radiale 25 du protège-aiguille 20. Avantageusement, cet organe de préhension externe 300 est réalisé sous la forme d'une broche ou clip en forme de U qui, après mise en place du capot externe 30 sur le protège-aiguille 20, vient s'insérer à travers des ouvertures 301 du capot externe 30 sous ladite projection radiale 25, comme visible sur la figure 14. Cette mise en oeuvre ajoute une pièce supplémentaire, mais rend l'assemblage du capot externe 30 moins complexe.

La mise en oeuvre d'une projection radiale 25 formée à l'extrémité axiale externe du protège-aiguille 20 qui coopère avec des moyens de préhension du capot externe 30 est particulièrement simple à fabriquer et à assembler, et particulièrement fiable en utilisation.

Les figures 15 et 16 montrent une variante de réalisation dans laquelle une bague 200 est ajoutée autour de la seringue 2, à proximité de l'extrémité avant 3 de ladite seringue. Cette bague 200 a pour objectif de coopérer avec le bord axial arrière de la structure rigide 28 du protège-aiguille 20. Cette bague 200 peut être un élément constitutif du dispositif d'injection. Elle peut comporter des doigts de maintien 201, dont le bord axial avant 208 va coopérer avec le protège-aiguille 20. Lors de la mise en place du capot externe 30 sur le protège-aiguille 20, ces doigts 201 permettent de bloquer ledit protège-aiguille en translation par butée mécanique. Ainsi, les efforts générés lors de la mise en place du capot externe sont directement transmis au niveau du contact entre la structure rigide 28 du protège-aiguille 20 et les doigts de maintien 201 de la bague 200. De cette manière, il ne se produit pas de déplacement du protège-aiguille 20 lors de la mise en place du capot externe, ce qui permet de conserver l'intégrité du dispositif, et d'éviter une détérioration de l'aiguille.

La figure 3 représente une variante de réalisation du dispositif des figures 1 et 2, dans lequel ledit épaulement radial 35 du capot externe 30 n'est pas formé de manière monobloc avec le capot 30, comme c'est notamment le cas dans le mode de réalisation des figures 1 et 2, mais il est formé sur une pièce mobile 39 qui est déplaçable à l'intérieur dudit capot externe 30.

Les figures 17 et 18 représentent un autre mode de réalisation qui n'est pas couvert par la présente invention, car le protège-aiguille ne comporte pas de structure externe rigide. Dans cette variante, le capot externe 30 a une forme plus complexe. Ce capot 30 des figures 17 et 18 pourrait néanmoins aussi s'appliquer à un protège-aiguille avec une structure externe rigide supportant la projection radiale distale 25, auquel cas il serait couvert par la présente invention.

Dans cette variante de réalisation, le capot externe 30 comporte un manchon interne 33 et un manchon externe 34. Le manchon externe 34 forme la partie extérieure du capot externe 30 alors que le manchon interne 33 coopère avec ledit protège-aiguille 20, notamment avec ladite projection radiale distale 25. Ledit manchon interne 33 du capot externe 30 peut être réalisé avec des pattes déformables 36 saillantes vers l'intérieur comme visible sur la figure 18, réparties autour de la périphérie dudit manchon interne. Ces pattes 36 peuvent notamment être formées au niveau d'ouvertures longitudinales ou axiales prévues dans ledit manchon interne 33.

Des pattes de stabilisation 37 du capot externe 30 par rapport à l'autoinjecteur peuvent être prévues entre le manchon externe 34 et le manchon interne 33, comme visible sur la figure 17.

D'autres modifications sont également possibles sans sortir du cadre de la présente invention tel que défini par les revendications annexées.

## Revendications

1. Dispositif d'injection comportant un système de protection d'aiguille, ledit dispositif d'injection comportant une aiguille de distribution (10) pourvue d'un orifice de distribution (11), ledit système de protection comportant un protège-aiguille amovible (20) adapté à être disposé, dans une position de non-distribution, autour de ladite aiguille (10) pour protéger et isoler ledit orifice de distribution (11) de ladite aiguille (10), ledit protège-aiguille (20) comportant un corps latéral creux (27) et une partie d'isolation (21), au moins ladite partie d'isolation (21) étant en matériau souple, ledit orifice de distribution (11) de l'aiguille (10) étant piqué dans ladite partie d'isolation (21) en position de non-distribution, **caractérisé en ce que** ledit protège-aiguille (10) comporte au moins une projection radiale vers l'extérieur (25), ledit système de protection comportant un capot externe (30) pourvu de moyens de préhension (35, 35', 36 ; 38 ; 300) adaptés à coopérer avec ladite au moins une projection radiale (25) dudit protège-aiguille (20), de telle sorte qu'un déplacement dudit capot externe (30) dans le sens longitudinal de ladite aiguille (10) en éloignement dudit orifice de distribution (11) retire ledit protège-aiguille (20) de sa position de non-distribution, ladite au moins une projection radiale (25) étant disposée à l'extrémité axiale distale dudit protège-aiguille (20), ladite au moins une projection radiale (25) étant formée sur une structure externe rigide (28) dudit protège-aiguille (20).

2. Dispositif selon la revendication 1 ou 2, dans lequel ladite au moins une projection radiale (25) est formée par un disque définissant une projection radiale périphérique.

3. Dispositif selon l'une quelconque des revendications précédentes, dans lequel lesdits moyens de préhension comportent au moins un épaulement radial (35, 35', 36) s'étendant radialement vers l'intérieur dudit capot externe (30) et coopérant, notamment par encliquetage, avec ladite au moins une projection radiale (25) dudit protège-aiguille (20).

4. Dispositif selon la revendication 3, dans lequel ledit au moins un épaulement radial est formé par une bride radiale périphérique (35) ou par plusieurs parties de bride radiale (35') réparties sur la périphérie.

5. Dispositif selon la revendication 3, dans lequel ledit au moins un épaulement est formé par une patte déformable (36).

6. Dispositif selon l'une quelconque des revendications précédentes, dans lequel lesdits moyens de préhension comportent une surface interne conique (38) coopérant par coincement et/ou frottements avec le bord radialement externe de ladite au moins une projection radiale (25).

7. Dispositif selon l'une quelconque des revendications précédentes, dans lequel lesdits moyens de préhension (35, 35', 36 ; 38) sont formés de manière monobloc avec ledit capot (30).

8. Dispositif selon l'une quelconque des revendications 1 à 6, dans lequel lesdits moyens de préhension (35, 35', 36) sont formés sur une pièce interne (39) mobile par rapport audit capot (30).

9. Dispositif selon l'une quelconque des revendications précédentes, dans lequel une bague (200) dudit dispositif d'injection coopère avec ledit protège-aiguille (20) en position de non-distribution pour former une butée mécanique lors de la mise en place du capot externe (30) autour dudit protège-aiguille (20).

10. Dispositif selon l'une quelconque des revendications précédentes, dans lequel ledit dispositif d'injection est un autoinjecteur.

## Patentansprüche

1. Injektionsvorrichtung, aufweisend ein Nadelschutzsystem, wobei die Injektionsvorrichtung eine Ausgabenadel (10) aufweist, die mit einer Ausgabeöffnung (11) versehen ist, wobei das Schutzsystem einen lösbaren Nadelschutz (20) aufweist, der dazu geeignet ist, in einer Nicht-Ausgabeposition um die Nadel (10) herum angeordnet zu werden, um die Ausgabeöffnung (11) der Nadel (10) zu schützen und zu isolieren, wobei der Nadelschutz (20) einen seitlichen Hohlkörper (27) und einen Isolationsteil (21) aufweist, wobei zumindest der Isolationsteil (21) aus einem flexiblen Material gefertigt ist, wobei die Ausgabeöffnung (11) der Nadel (10) in der Nicht-Ausgabeposition in den Isolationsteil (21) eingestochen ist, **dadurch gekennzeichnet, dass** der Nadelschutz (10) mindestens einen radialen Vorsprung (25) nach außen aufweist, wobei das Schutzsystem eine äußere Kappe (30) aufweist, die mit Greifmitteln (35, 35', 36; 38; 300) versehen ist, die dazu geeignet sind, mit dem mindestens einen radialen Vorsprung (25) des Nadelschutzes (20) derart zusammenzuwirken, dass eine Verschiebung der äußeren Kappe (30) in Längsrichtung der Nadel (10) sich von der Ausgabeöffnung (11) entfernend den Nadelschutz (20) aus seiner Nicht-Ausgabeposition abzieht, wobei der mindestens eine radiale Vorsprung (25) am axialen distalen Ende des Nadelschutzes (20) angeordnet ist, wobei der mindestens eine radiale Vorsprung (25) auf einer äußeren starren Struktur (28) des Nadelschutzes (20) ausgebildet ist.

2. Vorrichtung nach Anspruch 1 oder 2, wobei der mindestens eine radiale Vorsprung (25) durch eine Scheibe ausgebildet ist, die einen radialen Umfangsvorsprung bildet.

3. Vorrichtung nach einem der vorhergehenden Ansprüche, wobei die Greifmittel mindestens einen radialen Absatz (35, 35', 36) aufweisen, der sich radial ins Innere der äußeren Kappe (30) erstreckt und insbesondere durch Einrasten mit dem mindestens einen radialen Vorsprung (25) des Nadelschutzes (20)

4. Vorrichtung nach Anspruch 3, wobei der mindestens eine radiale Absatz durch einen radialen Umfangsflansch (35) oder durch mehrere radiale Flanschteile (35'), die um den Umfang verteilt sind, ausgebildet ist.

5. Vorrichtung nach Anspruch 3, wobei der mindestens eine Absatz durch eine verformbare Lasche (36) ausgebildet ist.

6. Vorrichtung nach einem der vorhergehenden Ansprüche, wobei die Greifmittel eine konische Innenfläche (38) aufweisen, die durch Verklemmung und/oder Reibung mit dem äußeren radialen Rand des mindestens einen radialen Vorsprungs (25) zusammenwirkt.

7. Vorrichtung nach einem der vorhergehenden Ansprüche, wobei die Greifmittel (35, 35', 36; 38) in einem Stück mit der Kappe (30) ausgebildet sind.

8. Vorrichtung nach einem der Ansprüche 1 bis 6, wobei die Greifmittel (35, 35', 36) auf einem inneren Teil (39), das relativ zur Kappe (30) beweglich ist, ausgebildet sind.

9. Vorrichtung nach einem der vorhergehenden Ansprüche, wobei ein Ring (200) der Injektionsvorrichtung in der Nicht-Ausgabeposition mit dem Nadelschutz (20) zusammenwirkt, um beim Anordnen der äußeren Kappe (30) um den Nadelschutz (20) einen mechanischen Anschlag auszubilden.

10. Vorrichtung nach einem der vorhergehenden Ansprüche, wobei die Injektionsvorrichtung selbstinjizierend ist.

## Claims

1. An injection device including a needle protection system, said injection device including a delivery needle (10) that is provided with a delivery orifice (11), said protection system comprising a removable needle guard (20) that, in a non-dispensing position, is adapted to be arranged around said needle (10) so as to protect and isolate said delivery orifice (11) of said needle (10), said needle guard (20) including a hollow lateral body (27) and an isolator portion (21), at least said isolator portion (21) being made of flexible material, said delivery orifice (11) of the needle (10) being poked into said isolator portion (21) in the non-dispensing position, the device being **characterized in that** said needle guard (10) includes at least one outwardly-radial projection (25), said protection system further comprising an outer cap (30) that is provided with grip means (35, 35', 36; 38; 300) that are adapted to co-operate with said at least one radial projection (25) of said needle guard (20), such that moving said outer cap (30) in the longitudinal direction of said needle (10), away from said delivery orifice (11), removes said needle guard (20) from the non-dispensing position, said at least one radial projection (25) being arranged at the distal axial end of said needle guard (20), said at least one radial projection (25) being formed on a rigid outer structure (28) of said needle guard (20).

2. A device according to claim 1 or claim 2, wherein said at least one radial projection (25) is formed by a disk that defines a peripheral radial projection.

3. A device according to any preceding claim, wherein said grip means comprise at least one radial shoulder (35, 35', 36) that extends radially towards the inside of said outer cap (30) and that co-operates, in particular by snap-fastening, with said at least one radial projection (25) of said needle guard (20).

4. A device according to claim 3, wherein said at least one radial shoulder is formed by a peripheral radial flange (35) or by a plurality of radial-flange portions (35') that are distributed over the periphery.

5. A device according to claim 3, wherein said at least one shoulder is formed by a deformable tab (36).

6. A device according to any preceding claim, wherein said grip means comprise a conical inside surface (38) that co-operates, by jamming and/or friction, with the radially-outer edge of said at least one radial projection (25).

7. A device according to any preceding claim, wherein said grip means (35, 35', 36; 38) are formed integrally with said cap (30).

8. A device according to any one of claims 1 to 6, wherein said grip means (35, 35', 36) are formed on an inner part (39) that is movable relative to said cap (30).

9. A device according to any preceding claim, wherein a ring (200) of said injection device co-operates with said needle guard (20) in the non-dispensing position so as to form a mechanical abutment while putting the outer cap (30) in place around said needle guard (20).

10. A device according to any preceding claim, wherein said injection device is an autoinjector.
